# EUROPEAN PATENT APPLICATION

(11) **EP 4 666 964 A2**
(43) Date of publication of application: **24.12.2025**
(21) Application number: 25183049.3
(22) Date of filing: 16.06.2025
(51) Int. Cl.: A61B 17/132

(54) **MULTIPURPOSE TOURNIQUET**

(30) Priority: 17.06.2024 KR 20240078310
(71) Applicant: Mediconny Co., Ltd., Namyangju-si, Gyeonggi-do, 12248 (KR); Park, Hyeon Soo, Daejeon 34812 (KR)
(72) Inventor: PARK, Hyeon Soo, 34812 Daejeon, (KR)
(74) Representative: Klunker IP Patentanwälte PartG mbB

(57) **Abstract**

To solve the problems described above, a multipurpose tourniquet, which includes a pressurizing portion that applies pressure to a puncture site and a dial portion including a central shaft axially coupled to one surface of the pressurizing portion and a handle spirally coupled to the central shaft to raise and lower the pressurizing portion, includes a support plate portion having one surface penetrated so that the central shaft is inserted thereinto, the support plate portion being seated on one surface of the pressurizing portion, and a band portion fixed by the handle which is seated on one surface of the support plate portion and spirally coupled to the central shaft.

## Description

### CROSS-REFERENCE TO RELATED APPLICATION

This application is based on and claims priority under 35 U.S.C. §119 to Korean Patent Application No. 10-2024-0078310, filed on June 17, 2024, in the Korean Intellectual Property Office, the disclosure of which is incorporated by reference herein in its entirety.

### BACKGROUND

### 1. Field

The present disclosure relates to a multipurpose tourniquet, and more particularly, to a multipurpose tourniquet in which a fastening portion extending in a semicircular shape is formed on a support plate portion to firmly bind a pressurizing portion and a band portion so as to firmly position the pressurizing portion that pressurizes a puncture site, and a buffer portion is formed on the pressurizing portion to buffer pressure applied to the pressurizing portion, thereby facilitating hemostasis.

### 2. Description of the Related Art

Generally, a vein is punctured when giving an injection into the human body, and an artery is punctured for procedures or blood gas tests.

Recently, many procedures have been performed through arteries. After procedures on radial artery or femoral artery, hemostasis is essential. Patients with kidney disease must have hemostasis after dialysis at an arterial or venous connection site.

That is, veins are mainly used for patient examination, nutrition administration, and drug administration, and arteries are used for almost all procedures. The procedure requires insertion of a tube after puncture. According to the size of the tube, the selection varies depending on the procedure, such as the wrist (radial artery), forearm, and groin.

Therefore, since hemostasis is possible only when there is a slight blood flow by varying the strength of the hemostasis, the hemostatic function must be different.

Additionally, when stopping bleeding after dialysis, if excessive pressure is applied to the swollen blood vessels, pain increases and eddies occur (that is, blood cannot flow and circulates in the blood vessels) to form blood clots, which may block the blood vessels.

In addition, when performing a coronary artery procedure on the heart using the radial artery or inguinal artery and then performing hemostasis, blood may not flow to other parts of the hand due to excessive pressure. After using a thrombolytic agent, long-term hemostasis is essential. However, if the pressure is too strong, a patient may suffer for a long time, and if the pressure is simply applied, skin necrosis may occur, which prevents hemostasis.

In addition, when the procedure is performed using the inguinal artery, a large tube is used and a clot-dissolving drug is overused, which makes the method of hemostasis important in terms of pressure and hemostasis site points.

That is, since the inguinal artery is in contact with the femoral region and a large tendon is formed over the blood vessel, the blood vessel is deep and more delicate actions are needed to apply pressure thereto. Therefore, in hemostasis of various arterial sites, it is very difficult to achieve the original purpose of hemostasis through simple pressure. In inguinal artery hemostasis, incorrect hemostasis causes a very dangerous problem that can be life-threatening because the artery is large and thus blood leakage occurs severely within the skin tissue.

To solve these problems, front, rear, and two side pressurizing members are needed, and the size of the pressurizing members should also be different for each site to facilitate hemostasis.

In particular, the hemostasis of the artery varies slightly from person to person, but hemostasis must be performed for 3 to 4 hours. In general, medical staff apply to hemostasis by using a hemostatic device that can stop bleeding or apply pressure by his/her hand.

Such conventional tourniquets stop bleeding by applying pressure to the surgical site with rolled gauze or by applying pressure to the surgical site with an inflated gauze. Recently, pad methods that guide hemostasis through chemical action have also been used.

The conventional hemostatic devices described above cannot achieve different hemostasis patterns depending on the site, resulting in various side effects.

For example, a large amount of blood may spurt between the puncture site and the skin, blood vessels may be deformed to form balloon-like vessels, or excessive pressure may be applied to the bleeding site, causing a blood clot.

In other words, the importance of hemostasis must be approached very seriously because patients are exposed to life-threatening risks due to excessive bleeding, which may lead to emergency surgery due to thrombosis or surgery due to abnormal development of blood vessels.

### Prior Art Documents

### Patent Documents

(Patent Document 0001) Korean Utility Model Registration No. 20-0470009 (registered on November 14, 2013)

### SUMMARY

To solve the problems of the related art, the present disclosure provides a multipurpose tourniquet having a novel structure in which a fastening portion extending in a semicircular shape is formed on a support plate portion to firmly bind a pressurizing portion and a band portion so as to firmly position the pressurizing portion that pressurizes a puncture site, and a buffer portion is formed on the pressurizing portion to buffer pressure applied to the pressurizing portion, thereby facilitating hemostasis.

In addition, the present disclosure provides a multipurpose tourniquet in which a support portion that is inclined downward on both sides of a support plate portion is formed to support a lower part of a band portion seated on one surface of the support plate portion.

In addition, the present disclosure provides a multipurpose tourniquet in which, when applying pressure to groin of a body and an puncture site adjacent to the groin of the body, a plurality of support unit plate portions are provided to respectively rotate in directions corresponding to the size and shape of the body to firmly wrap the body with the band portion.

In addition, the present disclosure provides a multipurpose tourniquet in which one side of an elastic band is formed to be shorter than the other side thereof, thereby allowing the two sides of the elastic band to be easily grounded to each other.

In addition, the present disclosure provides a multipurpose tourniquet in which, when the interior of a buffer holder is formed as a space and a central shaft pressurizes one side of the buffer holder, an angle of an inclined surface is lowered, and thus, pressure is distributed.

To solve the problems described above, a multipurpose tourniquet, which includes a pressurizing portion that applies pressure to a puncture site and a dial portion including a central shaft axially coupled to one surface of the pressurizing portion and a handle spirally coupled to the central shaft to raise and lower the pressurizing portion, includes a support plate portion having one surface penetrated so that the central shaft is inserted thereinto, the support plate portion being seated on one surface of the pressurizing portion, and a band portion fixed by the handle which is seated on one surface of the support plate portion and spirally coupled to the central shaft.

In an embodiment of the present disclosure, the support plate portion may include a support plate having a through hole with a center penetrating therethrough and a spiral formed on an inner circumferential surface of the through hole, and a fastening portion extending in a semicircular shape with an inclined surface on one surface of the support plate and forming a spiral on the inner circumferential surface so that the handle is spirally coupled.

In an embodiment of the present disclosure, the support plate portion may further include a support portion supporting the band portion seated on the support plate by slanting both sides of the support plate.

In an embodiment of the present disclosure, the band portion may include an elastic band forming a through hole on one surface so that central shaft is inserted into the through hole, and a binding portion forming a wider surface on both sides of the elastic band than a center and forming a separation hole spaced apart from a central portion along a length direction so as to be bound to a body in a puncture site in an X shape.

In an embodiment of the present disclosure, the band portion may be formed so that one side of the elastic band is shorter than another side of the elastic band.

In an embodiment of the present disclosure, a multipurpose tourniquet, which includes a pressurizing portion that applies pressure to a puncture site and a dial portion including a central shaft axially coupled to one surface of the pressurizing portion and a handle spirally coupled to the central shaft to raise and lower the pressurizing portion, includes a support unit plate portion comprising a plurality of unit plates having the center penetrated so that the central shaft is inserted thereinto, the plurality of unit plates being stacked and seated on one surface of the pressurizing portion, and a buffer portion that forms a buffer holder forming a downward slope from a lower side of the central shaft to both sides of the pressurizing portion and buffers pressure applied to the pressurizing portion.

In an embodiment of the present disclosure, the support unit plate portion may further include a unit fastening portion extending in a semicircular shape with an inclined surface on one surface of the unit plate on which the handle is seated, and forming a spiral on an inner surface so that the handle is spirally coupled thereto.

In an embodiment of the present disclosure, when the support unit plate portion pressurizes the pressurizing portion against groin of a body and a puncture site adjacent to the groin, the unit plate may be rotated and positioned according to a size and a shape of the body.

In an embodiment of the present disclosure, when the central shaft moves downward and pressurizes one surface of the buffer holder, an angle of the buffer holder may be lowered, so that the buffer portion distributes pressure.

In an embodiment of the present disclosure, the multipurpose tourniquet may further include a protrusion pressurizing portion in which a pressurizing protrusion protruding on one surface of the pressurizing portion and both sides of the pressurizing protrusion are formed in a curved shape.

### BRIEF DESCRIPTION OF THE DRAWINGS

FIG. 1 is a perspective view illustrating the exterior of a multipurpose tourniquet according to an embodiment of the present disclosure.
FIG. 2 is an exploded perspective view of FIG. 1.
FIG. 3 is a partially enlarged front view illustrating the multipurpose tourniquet according to an embodiment of the present disclosure.
FIG. 4 is an example diagram illustrating a state in which the multipurpose tourniquet according to an embodiment of the present disclosure is worn on an arm.
FIG. 5 is a perspective view illustrating a multipurpose tourniquet according to an application example of the present disclosure.
FIG. 6 is a plan view illustrating a state in which each of support unit plate portions of the multipurpose tourniquet according to the application example of the present disclosure is rotated.
FIG. 7 is an exploded perspective view of FIG. 5.
FIG. 8 is a side view illustrating a multipurpose tourniquet according to an application example of the present disclosure.

### DETAILED DESCRIPTION

Hereinafter, preferred embodiments will be described in detail with reference to the accompanying drawings, so that those of ordinary skill in the art can easily carry out the present disclosure. However, in describing the operating principle of a preferred embodiment of the present disclosure in detail, when the detailed description of the relevant known functions or configurations is determined to unnecessarily obscure the gist of the present disclosure, the detailed description thereof is omitted herein.

In addition, the same reference numerals are used to denote the parts that have similar functions and actions throughout the drawings.

In addition, it will be understood that, when a portion is referred to as being "connected to" another portion, it may be "directly connected to" the other portion or "indirectly connected to" the other portion with intervening elements therebetween. It will be understood that the terms "comprise" or "include" as used herein specify the presence of stated elements, but do not preclude the presence or addition of one or more other elements.

Hereinafter, a multipurpose tourniquet according to a preferred embodiment of the present disclosure will be described in detail with reference to the accompanying drawings.

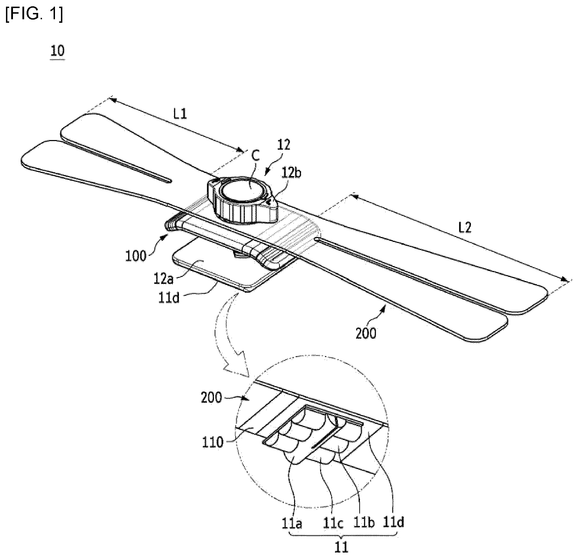

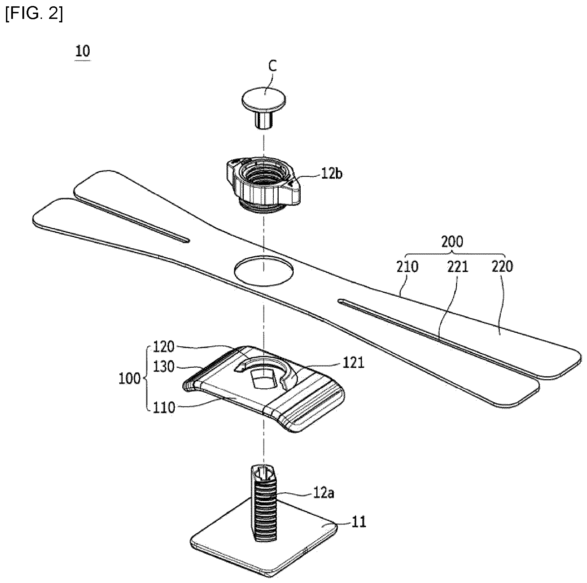

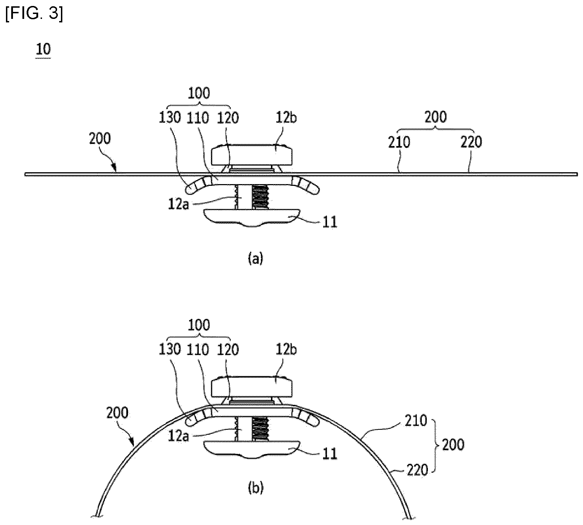

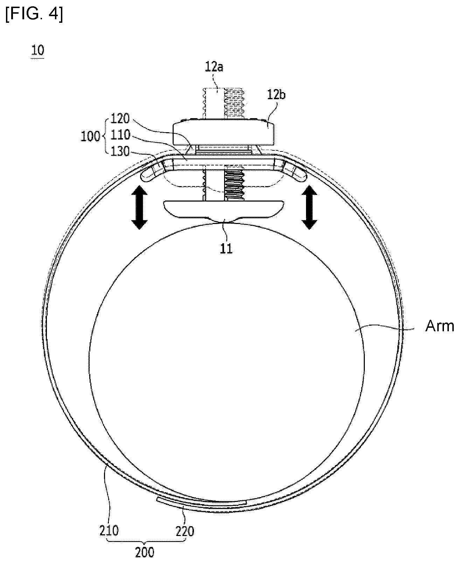

FIG. 1 is a perspective view illustrating the exterior of a multipurpose tourniquet according to an embodiment of the present disclosure, FIG. 2 is an exploded perspective view of FIG. 1, FIG. 3 is a partially enlarged front view illustrating the multipurpose tourniquet according to an embodiment of the present disclosure, and FIG. 4 is an example diagram illustrating a state in which the multipurpose tourniquet according to an embodiment of the present disclosure is worn on an arm.

As illustrated in FIG. 1, a multipurpose tourniquet 10 according to an embodiment of the present disclosure includes a support plate portion 100 and a band portion 200.

More specifically, as illustrated in FIGS. 1 and 2, the multipurpose tourniquet 10 according to an embodiment of the present disclosure, which includes a pressurizing portion 11 that applies pressure to a puncture site and a dial portion 12 including a central shaft 12a axially coupled to one surface of the pressurizing portion 11 and a handle 12b spirally coupled to the central shaft 12a to raise and lower the pressurizing portion 11, includes a support plate portion 100 having one surface penetrated so that the central shaft 12a is inserted thereinto, the support plate portion 100 being seated on one surface of the pressurizing portion 11, and a band portion 200 fixed by the handle 12b which is seated on one surface of the support plate portion 100 and spirally coupled to the central shaft.

As illustrated in FIG. 1, the pressurizing portion 11 includes a coupling plate 11d having one surface on which a support plate portion 100 is seated, a main pressurizing member 11a formed below the coupling plate 11d in a direction perpendicular to the band portion 200, center pressurizing members 11b extending on both sides of the center of the main pressurizing member 11a in a direction of the band portion 200, and front and rear pressurizing members 11c arranged on both sides of the center pressurizing member 11b in the same manner as the center pressurizing member 11b.

As used herein, the term "puncture site" indicates a site including a "bleeding site." Therefore, the "puncture site and bleeding site" are simply referred to as a "puncture site."

The pressurizing portion 11 pressurizes the main pressurizing member 11a closely to the puncture site, thereby enabling hemostasis at the puncture site. The center pressurizing members 11b apply pressure to the front and rear of the blood vessel with respect to the puncture site, and the front and rear pressurizing members 11c apply pressure to both sides of the front and rear of the puncture site with respect to the puncture site, thereby applying pressure to a wide area centered on the puncture site to achieve more effective hemostasis.

In addition, the dial portion 12 includes a central shaft 12a extending perpendicularly from the center of one surface of the coupling plate 11d, and a handle 12b that raises and lowers the support plate portion 100 that is spirally coupled to the central shaft 12a and is seated on one surface of the coupling plate 11d.

For the detailed description of the pressurizing portion 11 and the dial portion 12, see Korean Patent Registration No. 10-2654740 (registered on April 1, 2024) previously filed by the applicant.

On the other hand, referring to FIG. 1, the multipurpose tourniquet 10 according to an embodiment of the present disclosure may include a handle cap B coupled to one surface of the handle 12b.

The handle cap B may express logos and advertising text on one surface. The lower part of the handle cap B forms a protrusion that is spirally coupled to the upper part of the central shaft 12a, and the central shaft 12a forms a spiral hole in the inside corresponding to the protrusion on the upper inner side.

As illustrated in FIGS. 2 and 3, in the multipurpose tourniquet 10 according to the embodiment of the present disclosure, the support plate portion 100 is arranged between the pressurizing portion 11 and the band portion 200 so that the dial portion 12 increases the coupling force between the pressurizing portion 11 and the band portion 200.

To this end, the support plate portion 100 further includes a support plate 110, a fastening portion 120, and a support portion 130.

The support plate 110 is formed as a plate having a through hole with a center penetrating therethrough and having a spiral formed on the inner circumferential surface of the through hole, and the spiral formed on the outer circumferential surface of the central shaft 12a and the spiral formed on the inner circumferential surface of the through hole are coupled to each other and seated on one surface of the coupling plate 11d.

Referring to FIGS. 3 and 4, the fastening portion 120 extends in a semicircular shape with an inclined surface on one surface of the support plate 110 and forms a spiral on the inner circumferential surface so that the handle 12b is spirally coupled thereto.

More specifically, the fastening portion 120 forms a wall that surrounds the through hole formed on one surface of the support plate 110 in a semicircular shape and extends away from one surface of the support plate 110.

At this time, the wall forms an inclined surface 121 toward a distance spaced apart from one surface of the support plate 110, forms a spiral on the inner surface of the inclined surface 121, and is coupled to a spiral portion formed in the handle 12b. The fastening portion 120 is formed in a semicircular shape to facilitate the entry of the handle 12b that descends from the upper side of the central shaft 12a toward the other side.

That is, the handle 12b guides the entry into the inner surface of the inclined surface 121 to prevent detachment, and firmly seats the lower part of the handle 12b on one surface of the support plate 110. In addition, the lower part of the handle 12b is coupled only by a radius, which facilitates direction change.

In addition, when the central shaft 12a and the handle 12b are uncoupled and the handle 12b moves along the central shaft 12a, the upper part of the inclined surface 121 serves as a stopper to firmly fix the position of the handle 12b.

To this end, it is desirable that the lower part of the handle 12b is formed with a protrusion having a wider diameter than the spiral.

Referring to FIG. 4, the support portion 130 serves to support the band portion 200 seated on the support plate 110 by bending both sides of the support plate 110.

For example, when wearing the band portion 200 on the body part wrapping the arm, leg, thigh, or femoral region, one surface of the band portion 200 is bent at the edge of the support plate 110 while pulling the band portion 200 seated on the support plate 110. Since the bent portion may be damaged or stretched when worn for a long time, which may reduce the function of the band portion 200, it is desirable to form the support portion 130 that forms a slope on both sides of the support plate 110 to make the bent portion of the band portion 200 gentle.

As illustrated in FIGS. 1 and 2, in the multipurpose tourniquet 10 according to an embodiment of the present disclosure, the band portion 200 is a band that is seated on one surface of the support plate 110 and fixed by the handle 12b that is spirally coupled to the central shaft 12a.

To this end, the band portion 200 further includes an elastic band 210 and a binding portion 220.

The elastic band 210 wraps the body part where the puncture site occurs and enables the puncture site to be pressed by the pressurizing portion 11 coupled to the lower part of the support plate 110. In addition, a through hole corresponding to the support portion 130 is formed in one surface of the elastic band 210 so that the support portion 130 protrudes when seated on one surface of the support plate 110.

The binding portion 220 forms a wider surface on both sides of the elastic band 210 than the center and forms a separation hole 221 spaced apart from the central portion along the length direction so as to be bound to the body in the puncture site in an X shape.

That is, when the elastic band 210 is firmly worn on the body by making both sides wider than the center of the elastic band 210 and forming the separation hole 221 spacing the center of both sides of the elastic band 210 apart in the longitudinal direction, both sides of the elastic band 210 separated by the separation hole 221 may be overlapped perpendicular to each other or formed in an X shape.

As illustrated in FIG. 1, the band portion 200 is formed so that one side of the elastic band 210 is shorter than the other side of the elastic band 210.

That is, when a length L1 of one side of the elastic band 210 is made shorter than a length L2 of the other side of the elastic band 210 and the band portion 200 is worn on the body, either one side or the other side of the elastic band 210 is first brought into close contact with the body, and the other side is wrapped around the body and then grounded to one side.

For example, when the elastic band 210 is formed with the same length on both sides with respect to the center, there is an uncomfortable problem that one side flows down when the pressurizing portion 11 is seated on the puncture site so that one side is first brought into contact with the body and the other side is wrapped around the body and then grounded to one side.

Referring to FIG. 4, the multipurpose tourniquet 10 according to an embodiment of the present disclosure brings the pressurizing portion 11 into close contact with the puncture site and then rotates the handle 12b while wearing the band portion 200 on the body. The rotating handle 12b descends along the central shaft 12a and is fastened to the fastening portion 120, so that the central shaft 12a and the support plate 110 are more firmly attached and the support plate 110 pressurizes the pressurizing portion 11.

Due to this, the coupling plate 11d constituting the pressurizing portion 11 is adjacent to the puncture site, and the main pressurizing member 11a applies pressure on the puncture site. In addition, the center pressurizing member 11b and the front and rear pressurizing members 11c also apply pressure around the puncture site, thereby allowing hemostasis by applying pressure to a wide area with respect to the puncture site.

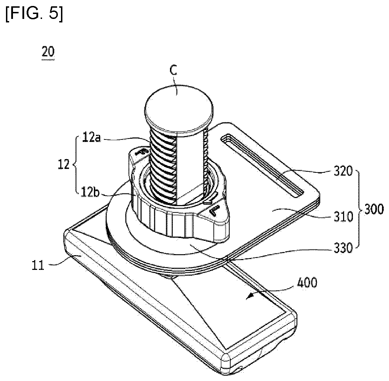

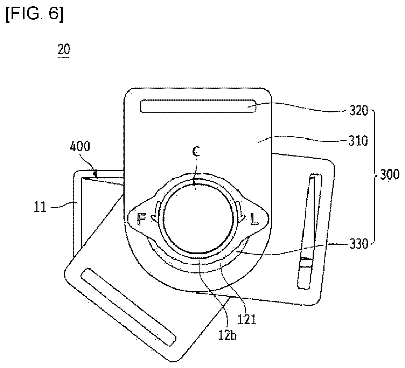

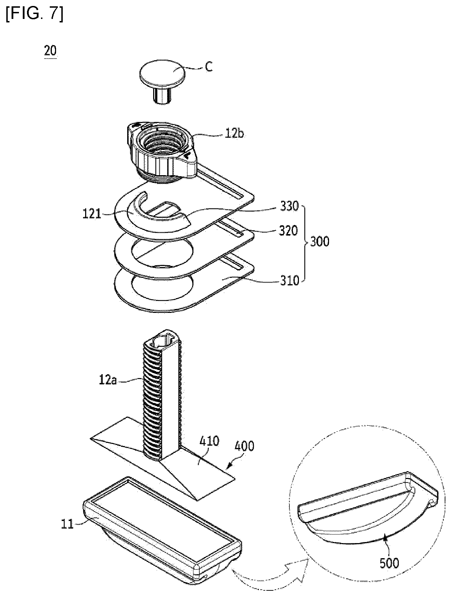

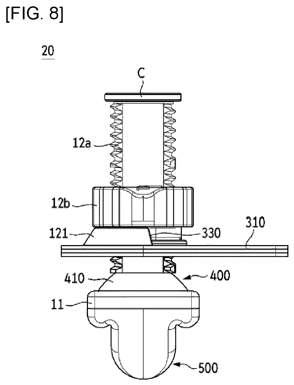

FIG. 5 is a perspective view illustrating a multipurpose tourniquet according to an application example of the present disclosure, FIG. 6 is a plan view illustrating a state in which each of support unit plate portions of the multipurpose tourniquet according to an application example of the present disclosure is rotated, FIG. 7 is an exploded perspective view of FIG. 5, and FIG. 8 is a side view illustrating a multipurpose tourniquet according to an application example of the present disclosure.

The multipurpose tourniquet 20 of the present disclosure described in the application example is formed by modifying the support plate portion to form a support unit plate portion 300 and adding a buffer portion 400 so that the groin and the puncture site adjacent to the groin of the body may be pressed by the pressurizing portion.

Referring to FIG. 5, the multipurpose tourniquet 20 of the present disclosure includes the support unit plate portion 300 and the buffer portion 400.

More specifically, as illustrated in FIGS. 5 and 6, the multipurpose tourniquet 20 according to an embodiment of the present disclosure, which includes a pressurizing portion 11 that applies pressure to a puncture site and a dial portion 12 including a central shaft 12a axially coupled to one surface of the pressurizing portion 11 and a handle 12b spirally coupled to the central shaft to raise and lower the pressurizing portion 11, includes a support unit plate portion 300 including a plurality of unit plates 310 having the center penetrated so that the central shaft 12a is inserted thereinto, the plurality of unit plates 310 being stacked and seated on one surface of the pressurizing portion 11, and a buffer portion 40 that forms a buffer holder 410 forming a downward slope from the lower side of the central shaft to both sides of the pressurizing portion and buffers the pressure applied to the pressurizing portion.

Referring to FIGS. 5 and 6, the support unit plate portion 300 may further include a unit plate 310, a coupling hole 320, and a unit fastening portion 330.

The unit plate 310 is a plate formed in a certain size to be rotatable around the central shaft 12a. One side of the unit plate 310 is formed in a curved shape so that the unit plate 310 is rotatable within a range that does not interfere with the groin.

That is, when the support unit plate portion 300 pressurizes the pressurizing portion 11 against the groin and the puncture site adjacent to the groin, the unit plate 310 may be rotated and positioned according to the size and shape of the body.

For example, since each person's body shape is different, the fixed belt and the fixed band are not suitable. Accordingly, it is desirable to form the belt and the fixing band in a direction that fits the body shape to facilitate direction change.

The coupling hole 320 is formed as a hole capable of binding a band to one side of the unit plate 310, and the unit plate 310 may be rotated in a desired direction to bring the pressurizing portion 11 into close contact with the puncture site by fastening the belt and the fixing band so as to wrap the body.

Referring to FIGS. 7 and 8, the unit fastening portion 330 extends in a semicircular shape with an inclined surface 121 on one surface of the unit plate 310 on which the handle 12b is seated, and forms a spiral on the inner surface so that the handle is spirally coupled thereto.

The unit fastening portion 330 is the same as the fastening portion 120 described above, the description of the fastening portion 120 is equally applied to the unit fastening portion 330.

In addition, the support unit plate portion 300 is formed in a circular shape and a reinforcing fastening portion 340 forming the inclined surface 121 is formed on the lower surface of the unit plate 310 so as to communicate with the unit fastening portion 330.

The reinforcing fastening portion 340 reinforces the lower part of the central shaft 12a when the central shaft 12a pressurizes the buffer holder 410 described below, thereby preventing distortion of the buffer holder 410.

Referring to FIGS. 7 and 8, the buffer portion 400 forms a downward buffer holder 410 that forms an inclination from the lower part of the central shaft 12a to both sides of the pressurizing portion 11 and serves to buffer the pressure applied to the pressurizing portion 11.

That is, when the central shaft 12a moves downward to press the buffer holder 410 of the buffer portion 400, the angle of the buffer holder 410 is lowered, thereby distributing the pressure.

At this time, the pressurizing portion 11 forms the coupling plate 11d illustrated in FIG. 1 in a rectangular shape that widens on both sides as illustrated in FIG. 7, and forms an accommodation portion that accommodates the buffer holder 410 on one surface. It is preferable to form double-sided pressurizing members on both sides of the main pressurizing member in the same direction as the main pressurizing member so as to be able to press the groin and the puncture site adjacent to the groin.

As illustrated in FIGS. 5 and 7, the multipurpose tourniquet 20 according to the application example of the present disclosure may include a protrusion pressurizing portion 500.

In the protrusion pressurizing portion 500, a pressurizing protrusion protruding on one surface of the pressurizing portion 11 and both sides of the pressurizing protrusion are formed in a curved shape.

For example, for the groin hemostasis site, various puncture procedures are performed on an artery located in a concave area where tendors, etc. are mixed therearound. Therefore, a horizontal and vertical hemostatic support to be applied to the wrist, etc., is formed in a straight-line shape, a central block of the straight-line support is expanded, and blocks on both sides assist the central block to receive constant pressure, thereby preventing the pressure from being distributed to the surroundings.

The multipurpose tourniquet according to the present disclosure has an advantage of facilitating hemostasis while reducing the burden on blood vessels by adjusting the pressure of the pressurizing portion that applies pressure to the front, back, and sides of the puncture site.

In addition, there is an advantage in that the fastening portion extending in a semicircular shape is formed on the support plate portion seated on the pressurizing portion, thereby facilitating the entry of the handle, and the coupling force between the handle and the support plate portion is reinforced, thereby preventing the handle from being detached.

**In** addition, there is an advantage in that, when applying pressure to the groin of the body and the puncture site adjacent to the groin of the body, the plurality of support unit plate portions are provided to respectively rotate in directions corresponding to the size and shape of the body to firmly wrap the body with the band portion.

**In** addition, for hemostasis of the wrist and other parts, one side of the elastic band is formed to be shorter than the other side thereof, thereby allowing the two sides of the elastic band to be easily grounded to each other. Therefore, even non-experts may easily perform medical procedures.

In addition, when the interior of the buffer holder is formed as a space and the central shaft pressurizes one surface of the buffer holder, the angle of the inclined surface is lowered, and thus, pressure is distributed. Therefore, there is an advantage in that the pressure applied to the puncture site may be adjusted to facilitate hemostasis.

As described above, preferred embodiments of the present disclosure have been described in detail, but this is an example of the best mode of the present disclosure and is not intended to limit the present disclosure. In addition, it will be understood by those of ordinary skill in the art that various modifications and changes can be made without departing from the scope of the technical idea of the present disclosure.

Therefore, the scope of the present disclosure is not limited to the embodiments described above, but can be implemented in various forms of embodiments within the scope of the appended claims. Furthermore, it will be understood by those of ordinary skill in the art that various modifications fall within the scope of the claims of the present disclosure without departing from the gist of the present disclosure claimed in the claims.

### Explanation of Reference Numerals

10, 20: multipurpose tourniquet
11: pressurizing portion
11a: main pressurizing member
11b: center pressurizing member
11c: front and rear pressurizing member
11d: coupling plate
12: dial portion
12a: central shaft
12b: handle
100: support plate portion
110: support plate
120: fastening portion
121: inclined surface
130: support portion
200: band portion
210: elastic band
220: binding portion
221: separation hole
300: support unit plate portion
310: unit plate
320: coupling hole
330: unit fastening portion
340: reinforcing fastening portion
400: buffer portion
410: buffer holder
500: protrusion pressurizing portion

## Claims

1. A multipurpose tourniquet, which includes a pressurizing portion that applies pressure to a puncture site and a dial portion including a central shaft axially coupled to one surface of the pressurizing portion and a handle spirally coupled to the central shaft to raise and lower the pressurizing portion, the multipurpose tourniquet comprising:
a support plate portion having one surface penetrated so that the central shaft is inserted thereinto, the support plate portion being seated on one surface of the pressurizing portion; and
a band portion fixed by the handle which is seated on one surface of the support plate portion and spirally coupled to the central shaft.

2. The multipurpose tourniquet of claim 1, wherein the support plate portion comprises:
a support plate having a through hole with a center penetrating therethrough and a spiral formed on an inner circumferential surface of the through hole; and
a fastening portion extending in a semicircular shape with an inclined surface on one surface of the support plate and forming a spiral on the inner circumferential surface so that the handle is spirally coupled.

3. The multipurpose tourniquet of claim 2, wherein the support plate portion further comprises a support portion supporting the band portion seated on the support plate by slanting both sides of the support plate.

4. The multipurpose tourniquet of claim 1, wherein the band portion comprises:
an elastic band forming a through hole on one surface so that central shaft is inserted into the through hole; and
a binding portion forming a wider surface on both sides of the elastic band than a center and forming a separation hole spaced apart from a central portion along a length direction so as to be bound to a body in a puncture site in an X shape.

5. The multipurpose tourniquet of claim 4, wherein the band portion is formed so that one side of the elastic band is shorter than another side of the elastic band.

6. A multipurpose tourniquet, which includes a pressurizing portion that applies pressure to a puncture site and a dial portion including a central shaft axially coupled to one surface of the pressurizing portion and a handle spirally coupled to the central shaft to raise and lower the pressurizing portion, the multipurpose tourniquet comprising:
a support unit plate portion comprising a plurality of unit plates having the center penetrated so that the central shaft is inserted thereinto, the plurality of unit plates being stacked and seated on one surface of the pressurizing portion; and
a buffer portion that forms a buffer holder forming a downward slope from a lower side of the central shaft to both sides of the pressurizing portion and buffers pressure applied to the pressurizing portion.

7. The multipurpose tourniquet of claim 6, wherein the support unit plate portion further comprises a unit fastening portion extending in a semicircular shape with an inclined surface on one surface of the unit plate on which the handle is seated, and forming a spiral on an inner surface so that the handle is spirally coupled thereto.

8. The multipurpose tourniquet of claim 6, wherein, when the support unit plate portion pressurizes the pressurizing portion against groin of a body and a puncture site adjacent to the groin, the unit plate is rotated and positioned according to a size and a shape of the body.

9. The multipurpose tourniquet of claim 6, wherein, when the central shaft moves downward and pressurizes one surface of the buffer holder, an angle of the buffer holder is lowered, so that the buffer portion distributes pressure.

10. The multipurpose tourniquet of claim 6, further comprising a protrusion pressurizing portion in which a pressurizing protrusion protruding on one surface of the pressurizing portion and both sides of the pressurizing protrusion are formed in a curved shape.
